# EUROPÄISCHE PATENTSCHRIFT

(11) **EP 0 977 529 B1**
(45) Veröffentlichungstag und Bekanntmachung des Hinweises auf die Patenterteilung: **12.02.2003**
(21) Anmeldenummer: 99910246.0
(22) Anmeldetag: 19.02.1999
(51) Int. Cl.: A61F 2/44

(54) **ZWISCHENWIRBELIMPLANTAT**
INTERVERTEBRAL IMPLANT
IMPLANT INTERVERTEBRAL

(30) Priorität: 20.02.1998 DE 19807236
(43) Veröffentlichungstag der Anmeldung: 09.02.2000
(73) Patentinhaber: BIEDERMANN MOTECH GmbH, 78054 Villingen-Schwenningen (DE)
(72) Erfinder: HARMS, Jürgen, D-76337 Waldbronn (DE); BIEDERMANN, Lutz, D-78048 VS-Villingen (DE); WICHMANN, Thomas, D-77716 Hofstetten (DE)
(74) Vertreter: Prüfer, Lutz H., Dipl.-Phys.
(86) Internationale Anmeldenummer: EP9901099
(87) Internationale Veröffentlichungsnummer: WO99042062

(56) Entgegenhaltungen:
- EP-A- 0 635 246
- WO-A-97/00054
- WO-A-97/06753
- WO-A-97/15248
- WO-A-98/48739
- DE-A- 3 608 163
- DE-A- 4 447 057
- FR-A- 2 717 068
- US-A- 5 522 899
- US-A- 5 609 635
- US-A- 5 658 335

## Beschreibung

Die Erfindung betrifft ein Zwischenwirbelimplantat mit zwei einen Abstand zueinander aufweisenden Seitenwänden, einer diese an ihrem einen Ende verbindenden Vorderwand, einer diese an ihrem gegenüberliegenden anderen Ende verbindenden Rückwand und je einer Öffnung auf den sich quer zu den vorgenannten Wänden erstreckenden Boden- und Deckflächen.

Ein solches Implantat wird nach der Entfernung einer Bandscheibe eingesetzt, um den Zwischenwirbelraum zu stabilisieren, bis das gleichzeitig eingebrachte Knochenmaterial zu einer knöchernen Durchbauung und Versteifung geführt hat.

Ein Zwischenwirbelimplantat ist aus der DE 195 29 605 C2 bekannt. Das Implantat weist zwei zunächst in einen Hohlraum zurückgebogene hakenförmige Abschnitte auf, die durch Einschrauben einer einen kegelförmigen Abschnitt aufweisenden Schraube nach Einsetzen des Implantates zwischen zwei Wirbelkörper nach außen in eine Greifstellung bewegt werden. Ein Nachteil dieser Vorrichtung besteht darin, daß die die Greifer aufweisenden Arme nicht in ihre zurückgezogene Stellung zurückbewegbar sind, so daß ein Verändern der Position des Implantates durch den Operateur nach dem ersten Ausfahren der Greifer nicht mehr möglich ist.

Aus der DE 195 49 426 C2 ist ein Zwischenwirbelimplantat bekannt, bei dem zwei Endabschnitte eines Hohlkörpers durch Einsetzen einer einen kegelstumpfförmigen Abschnitt aufweisenden Schraube diese aus einer zunächst zusammengebogenen Stellung in eine auseinandergedrückte Stellung bewegt werden. Auf der Außenseite vorgesehene Zacken greifen in der auseinandergedrückten Stellung in die benachbarten Wirbelkörper ein. Auch hier besteht der Nachteil, daß nach dem erstmaligen Auseinanderdrücken und Eingreifen der Zacken in die benachbarten Wirbelkörper keine Rückführung zum Ersetzen oder Nachjustieren möglich ist.

Aus der US 5,522,899 ist ein Zwischenwirbelimplantat bekannt. Dieses umfaßt ein Unterteil aus einem rechteckigen Boden mit sich dazu im rechten Winkel erstreckenden zwei Seitenwänden, die rechteckig ausgebildet sind und in ihrem mittleren Bereich jeweils eine vom Boden sich weg erstreckende hervorstehende Zunge aufweisen. Ferner ist ein Oberteil vorgesehen, welches einen entsprechenden rechteckigen Boden und zwei zu dem Unterteil hin sich im rechten Winkel zum Boden erstreckende Seitenwände aufweist, die jeweils einen Abschnitt zur Aufnahme der Zungen des Unterteiles aufweisen. Die jeweils einander zugewandten Seiten der Böden sind keilsegmentförmig ausgebildet und so bemessen, daß zwischen den einander zugewandten Böden ein Freiraum gebildet ist. In diesem Freiraum sind zwei mit ihrer Basis voneinander abgewandte Keile vorgesehen, die zueinander konzentrische Bohrungen aufweisen. Die Bohrung des ersten Keiles ist gewindelos und dient als Führung für eine Schraube, während die Bohrung des zweiten Keiles ein Innengewinde aufweist, welches dem Gewinde der Schraube entspricht.

Die Länge der Schraube ist so gewählt, daß sie durch den ersten Keil hindurchgeführt in den zweiten Teil eingeschraubt ist, wenn die beiden Keile sich in ihrem größten Abstand befinden, in dem die beiden keilförmigen Elemente in ihrem kleinsten Abstand voneinander angeordnet sind. Durch Drehen der Schraube wird der zweite Keil in Richtung zum ersten Keil gezogen, so daß dadurch der Abstand der beiden keilförmigen Elemente sich verkleinert. Als Ergebnis tritt aus dem zweiten Keil das freie Ende der Schraube heraus. Da die Schraube selbst in ihrer Lagen zu den übrigen Elementen nicht geführt ist, ist auch die Bewegung der beiden Teile relativ zu den übrigen Elementen nicht kraft- bzw. formschlüssig vorbestimmt.

In der US 5,522,899 wird ein weiteres Ausführungsbeispiel beschrieben, bei dem das Zwischenwirbelimplantat ein generell rechteckiges Gehäuse mit zwei Seitenwänden, je einer Vorderund Rückwand und je einer Deck- und Bodenwand aufweist. Deckund Bodenwand weisen jeweils kleine Ausnehmungen auf. Im Inneren des so geformten Gehäuses sind Krallen vorgesehen, die so schwenkbar gelagert sind, dass sie von einer in das Innere des Gehäuses zurückgezogenen Stellung in eine zweite Stellung bewegbar sind, in der die Spitzen der Krallen durch die Öffnungen nach außen vorstehen. Das Schwenken der Krallen wird mittels zweier Keile und einer Schraube in einer Weise durchgeführt, die der des zuvor erläuterten Ausführungsbeispiels entspricht. Durch Drehen der Schraube wird der Abstand der beiden Keile verringert. Dadurch werden die Schwenkteile nach außen getrieben. Auch hier liegt keine kraft- bzw. formschlüssige Bewegung der beiden Teile vor. Der Abstand der mit den benachbarten Wirbelkörpern in Eingriff bringbaren Deck- und Bodenwand ist konstant.

Weiterhin ist aus der US 5,658,335 ein einstellbares Zwischenwirbelimplantat bekannt, das zwei Seitenwände aufweist, die ungefähr in der Mitte über eine Zwischenwand miteinander verbunden sind. Durch diese Zwischenwand ist ein Kernelement mit Außengewinde geführt, das durch einen Flansch gegen völliges Durchrutschen gesichert ist. Das Kernelement hat zwei Gewindeabschnitte mit entgegengesetzt gerichteter Steigung, auf denen je eine Mutter mit Innengewinde aufgeschraubt sind. Die nach innen weisenden Flächen dieser Muttern sind abgeschrägt. An diesen Schrägen liegen vier Kappenelemente an, die mit Zacken versehen sind. Beim Drehen des Kernelements werden die Muttern nach innen gezogen und treiben ihrerseits die Kappenelemente nach außen, so dass die Zacken in die benachbarten Wirbel eingreifen. Durch die fehlende Lagerung des Kernelements, das durch die Zwischenwand nur hindurchgeführt, aber nicht darin gelagert ist, ist dieser Aufbau jedoch sehr instabil.

Aufgabe der Erfindung ist es, ein verbessertes Zwischenwirbelimplantat zu schaffen, das die Nachteile des angeführten Stands der Technik überwindet.

Diese Aufgabe wird durch das im Patentanspruch 1 gekennzeichnete Zwischenwirbelimplantat gelöst.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen:
- Fig. 1: eine perspektivische Ansicht eines ersten Ausführungsbeispieles eines Zwischenwirbelimplantates in Explosionsdarstellung;
- Fig. 2: einen Teil einer Wirbelsäule mit dem eingesetzten Zwischenwirbelimplantat in Seitenansicht;
- Fig. 3: ein Detail aus Fig. 2 in vergrößertem Maßstab;
- Fig. 4: eine Draufsicht auf das Zwischenwirbelimplantat;
- Fig. 5: eine Seitenansicht des Zwischenwirbelimplantats;
- Fig. 6: eine stirnseitige Ansicht des Zwischenwirbelimplantats;
- Fig. 7: eine Schnittansicht durch das Zwischenwirbelimplantat entlang der Linie IV-IV von Fig. 6 mit den Zacken in zurückgezogener Position;
- Fig. 8: eine Schnittansicht durch das Zwischenwirbelimplantat entlang der Linie IV-IV von Fig. 6 mit den Zacken in hervorstehender Position;
- Fig. 9: eine der Fig. 7 entsprechende Schnittansicht einer zweiten Ausführungsform; und
- Fig. 10: eine der Fig. 8 entsprechende Schnittdarstellung der zweiten Ausführungsform.

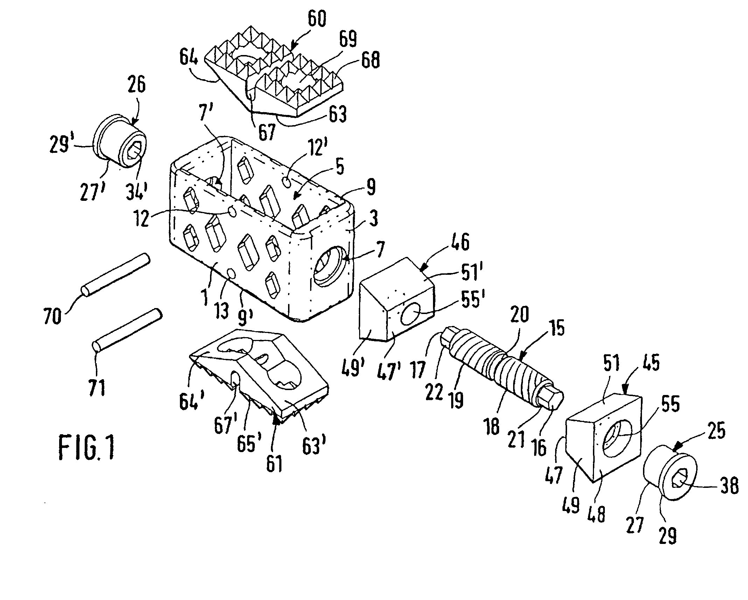

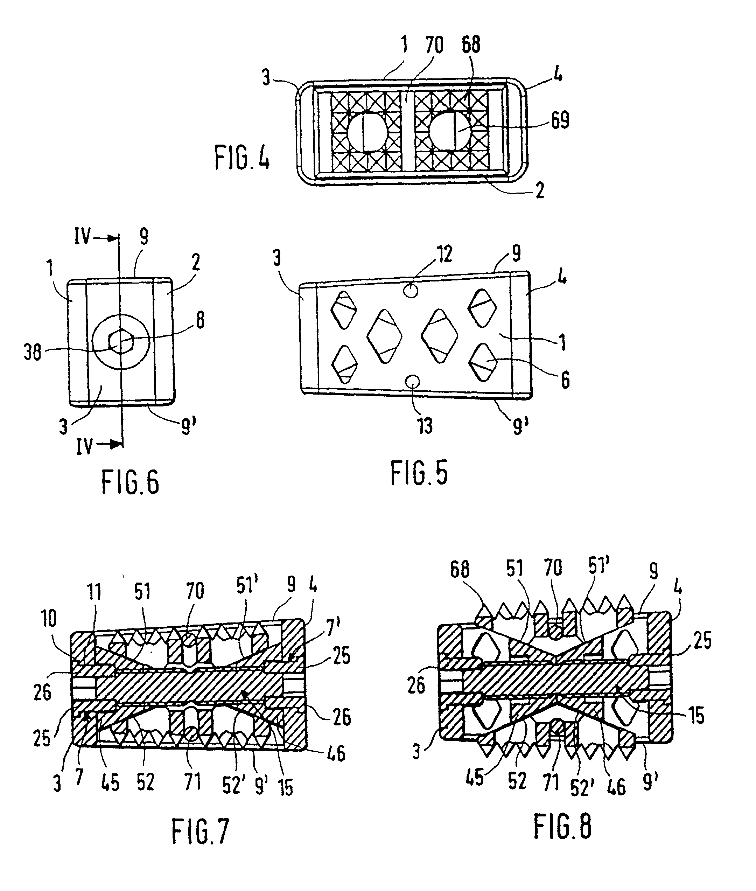

Wie am besten aus den Figuren 1 und 4 bis 6 ersichtlich ist, weist das Implantat eine erste Seitenwand 1, eine dieser in einem Abstand gegenüberliegende zweite Seitenwand 2 und eine die beiden Seitenwände 1,2 an ihrem einen Ende verbindende Vorderwand 3 sowie eine dieser gegenüberliegende Rückwand 4 zum Verbinden der beiden Seitenwände an ihren gegenüberliegenden Enden auf. Die Boden- und Deckflächen sind, wie am besten aus Fig. 1 ersichtlich ist, offen, so daß die vier Wände einen oben und unten offenen Hohlraum 5 umschließen. Die beiden Seitenwände 1 und 2 weisen, wie am besten aus Fig. 5 ersichtlich ist, eine Mehrzahl von über die Wandflächen verteilte Ausnehmungen 6 auf, die vorzugsweise rhombenartig ausgeführt sind.

Wie am besten aus den Figuren 1 und 6 bis 8 ersichtlich ist, ist in der Vorderwand 3 und der Rückwand 4 in der Mitte zwischen den Seitenwänden jeweils eine koaxial zu der jeweils anderen Bohrung ausgerichtete Bohrung 7, 7' vorgesehen. Der Mittelpunkt 8 der Bohrung 7, 7' befindet sich jeweils in gleichem Abstand von dem oberen bzw. unteren Rand 9, 9'des Implantats und somit im Zentrum der Vorder- bzw. Rückwand. Die Bohrung 7, 7' weist einen ersten an der Außenseite der Vorder- bzw. Rückwand gelegenen Bereich 10 mit einem ersten Durchmesser auf und einen daran angrenzenden in den Hohlraum 5 mündenden zweiten Bereich 11 mit einem zweiten Durchmesser auf, wobei der zweite Durchmesser etwas kleiner ist als der erste Durchmesser. Dadurch bildet der zweite Bereich 11 eine Schulter.

Jede Seitenwand 1, 2 weist in etwa mittig zwischen der Vorderund Rückwand angeordnet und in einem Abstand von dem oberen und unteren freien Rand 9, 9' des Implantats vorgesehene Bohrungen 12, 13 bzw. 12', 13' auf, wobei jeweils gegenüberliegende Bohrungen 12, 12' bzw. 13, 13' der Seitenwände 1, 2 koaxial zueinander sind.

In das Paar koaxialer Bohrungen 7, 7' ist, wie am besten aus den Figuren 1 und 7 und 8 ersichtlich ist, eine Gewindespindel 15 eingesetzt. Die Gewindespindel 15 weist ein erstes Ende 16 und ein diesem gegenüberliegendes zweites Ende 17 auf. Zwischen dem ersten Ende 16 und dem zweiten Ende 17 ist ein sich jeweils von in einem Abstand von dem ersten Ende 16 bis knapp zur Mitte 20 der Gewindespindel erstreckender erster Gewindeabschnitt 18 und ein sich in einem Abstand vom zweiten Ende 17 bis knapp zur Mitte 20 der Gewindespindel erstreckender zweiter Gewindeabschnitt 19 vorgesehen. Die Gewindesteigung des ersten Gewindeabschnittes 18 ist entgegengesetzt zu der Gewindesteigung des zweiten Gewindeabschnittes 19. Angrenzend an das erste Ende 16 bzw. das zweite Ende 17 weist die Gewindespindel jeweils einen ersten Sechskant 21 bzw. einen zweiten Sechskant 22 auf.

Wie insbesondere aus den Figuren 6 bis 8 ersichtlich ist, ist jedes Ende 16, 17 der Gewindespindel 15 in ein entsprechendes Lagerelement (bzw. einen Lagerzapfen) 25, 26 drehfest eingeführt, welches seinerseits jeweils in den Bohrungen 7, 7' drehbar gelagert ist und dessen Außenfläche mit der Vorder- bzw. Rückwand bündig abschließt. Jedes Lagerelement 25, 26 ist als Teil mit einem ersten zylinderförmigen Abschnitt 27, 27' mit einem ersten Außendurchmesser und einem daran angrenzenden zylinderförmigen zweiten Abschnitt 29, 29' mit einem zweiten Außendurchmesser, wobei der zweite Außendurchmesser größer als der erste Außendurchmesser ist, ausgebildet. Dadurch bildet der zweite Abschnitt 29, 29' jeweils einen über den ersten Abschnitt 27, 27' hervorstehenden ringförmigen Ansatz. Die Durchmesser der ersten und zweiten Abschnitte sind so gewählt, daß das Lagerelement 25 bzw. 26 in die Bohrung 7, 7' der Vorder- bzw. Rückwand einschiebbar ist, und daß in eingeschobenem Zustand der durch den zweiten Bereich gebildete Ansatz des Lagerteils auf der Schulter der Bohrung 7, 7' aufliegt. Der erste Abschnitt 27 bzw. 27' des Lagerteiles 25 bzw. 26 weist eine sich von dem dem zweiten Abschnitt 29 bzw. 29' gegenüberliegenden Ende in das Innere des zylinderförmigen Abschnittes 27 bzw. 27' erstreckende Sechskantbohrung 34 bzw. 34' auf, deren Längsabmessung der Abmessung des Endabschnittes 21 bzw. 22 der Gewindespindel 15 entspricht und in die auch die Endbereiche 21 bzw. 22 der Gewindespindel 15 jeweils paßgenau eingeführt sind. Von dem jeweils gegenüberliegenden Ende eines jeden Lagerteils 25, 26 erstreckt sich eine koaxiale Sechskantbohrung 38, 38' in Richtung des ersten Abschnittes 27, 27' zum Ineingriffbringen mit einem Sechskant-Schraubendreher. Dadurch wird ermöglicht, daß ein Operateur von beiden Seiten an das Implantat herankommt und dieses justieren kann.

Wie insbesondere aus den Figuren 1, 7 und 8 ersichtlich ist, befindet sich auf jedem Gewindeabschnitt 18, 19 der Gewindespindel 15 ein keilförmiges Element 45 bzw. 46. Das keilförmige Element 45 bzw. 46 ist jeweils begrenzt von einer Vorderfläche 47, 47' und einer Rückfläche 48, 48' mit rechteckigem Querschnitt, die parallel zueinander und senkrecht zur Spindelachse verlaufen, wobei die Vorderfläche 47, 47' einen kleineren Querschnitt aufweist als die Rückfläche 48, 48'. Vorderfläche und Rückfläche sind über zwei einander gegenüberliegende trapezförmige Seitenflächen 49, 49' und jeweils eine Deck- bzw. Bodenfläche 51, 51' bzw. 52, 52' mit rechteckigem Querschnitt verbunden, wodurch ein Keil gebildet ist.

Das keilförmige Element 45 weist eine jeweils durch die Mitte der Vorder- und Rückfläche hindurchgehende Gewindebohrung 55 mit einem dem Außengewinde des Gewindeabschnitts 18 der Gewindespindel 15 entsprechenden Innengewinde auf. Das keilförmige Element 46 weist ebenfalls eine durch die Mitte der Vorderund Rückfläche hindurchgehende Gewindebohrung 55 mit einem dem Außengewinde des Gewindeabschnitts 19 der Gewindespindel 15 entsprechenden Innengewinde auf. Die keilförmigen Elemente 45 und 46 sind so auf die jeweiligen Gewindeabschnitte 18 bzw. 19 der Gewindespindel 15 aufgeschraubt, daß ihre jeweiligen Deckflächen 51, 51' und ihre jeweiligen Bodenflächen 52, 52' gegeneinander zugeneigt sind.

Zwischen die gegeneinander geneigten Deckflächen 51. 51' bzw. zwischen die gegeneinander geneigten Bodenflächen 52, 52', im folgenden als Keilflächen bezeichnet, der keilförmigen Elemente 45, 46 ist jeweils ein Element 60 bzw. 61 eingesetzt, welches an seiner den keilförmigen Elementen zugewandten Unterseite in Form eines Dachgiebels mit zwei schräg aufeinander zulaufenden Flächen 63, 64 bzw. 63', 64' ausgebildet ist. Der Neigungswinkel der Flächen 63, 64 bzw. 63', 64' entspricht dem Keilwinkel der keilförmigen Elemente. An seiner der Gewindespindel 15 abgewandten Seite weist jedes Eingriffsteil 60 bzw. 61 eine Oberfläche mit rechteckigem Umriß auf. In Längsrichtung zwischen Vorder- und Rückwand 3, 4 gesehen in der Mitte weist jedes Element einen sich parallel zur Vorder- bzw. Rückwand 3 und senkrecht zur Oberfläche des Elementes erstreckenden U-förmigen Schlitz 67 auf, dessen Grund zur Unterseite des Elementes hin gerichtet ist. Die Oberflächen weisen Zacken 68 auf, die jeweils auf dem Umriß eines Quadrates angeordnet sind, wie insbesondere aus Fig. 1 und Fig. 4 ersichtlich ist. Zwischen den Zacken befindet sich jeweils eine in etwa kreisförmige Vertiefung 69, so daß die auf dem Umriß eines Quadrates angeordneten Zacken jeweils einen Zackenkranz bilden.

In der Mitte zwischen Vorder- und Rückwand 3, 4 erstreckt sich jeweils in einem vorgegebenen Abstand von Boden- und Deckfläche ein parallel zur Vorder- und Rückwand sich erstreckender Stift 70, 71, der in den sich gegenüberliegenden Seitenwänden festgehalten ist. Der Durchmesser der Stifte 70, 71 ist etwas geringer als der Durchmesser des U-förmigen Schlitzes 67. Wie aus den Figuren ersichtlich ist, sitzt der Stift in dem U-förmigen Schlitz 70, 71 und bildet somit eine Führung für die bewegbaren Elemente und mit seinem Grund gleichzeitig einen Anschlag zur Begrenzung der Bewegung der Elemente nach außen. Die Anordnung der Stifte 70, 71 und die Tiefe der U-förmigen Schlitze 70, 71 sind so aufeinander abgestimmt, daß durch die relative Lage von Stift und Tiefe des Schlitzes die maximale Höhe des Herausfahrens des jeweiligen Elementes über die Boden- und Deckfläche hinaus bestimmt ist.

Die Abmessungen der keilförmigen Elemente 45, 46, der Gewindeabschnitte 18, 19, der Spindel 15 und der Eingriffsteile 60, 61, sowie die Steigung der Gewinde ist so gewählt, daß die Eingriffsteile 60, 61 von einer ersten Stellung, die in Fig. 7 dargestellt ist, in der sich die Zacken 68 unterhalb des Randes 9, 9' befinden, und einer zweiten Stellung, die in Fig. 8 gezeigt ist, in der die Zacken über den jeweiligen Rand 9, 9' des Implantats hinausragen bewegt werden können.

Das Implantat ist aus einem körperfreundlichen Material wie beispielsweise Titan gefertigt.

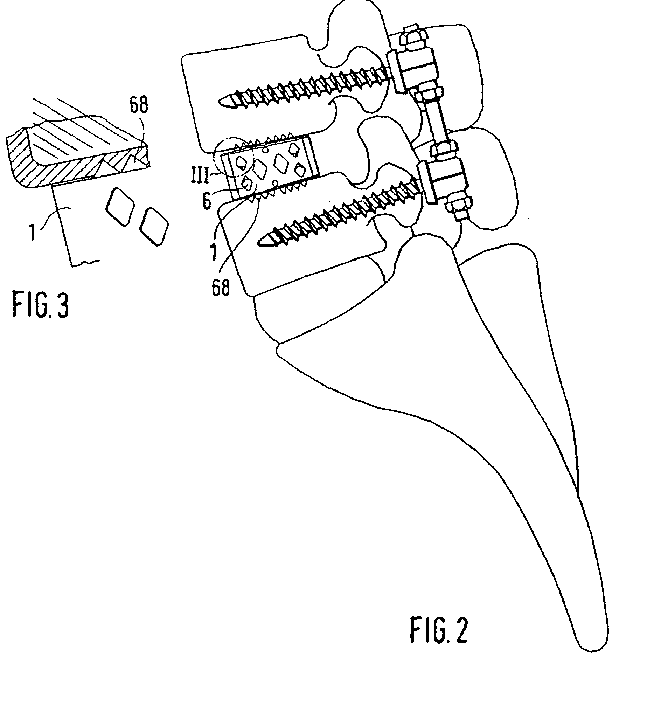

Im Betrieb werden mit einem Sechskant-Schraubendreher zunächst die keilförmigen Elemente 45, 46 durch Drehen der Gewindespindel 15 in die in Fig. 7 gezeigte Stellung gebracht, bei der die Rückflächen 48 an der dem Hohlraum 5 zugewandten Seite der Vorder- bzw. Rückwand 3, 4 des Implantates anliegen. Dadurch befindet sich jedes Eingriffsteil 60, 61 in seiner tiefsten Stellung, in der die Zacken 68 nicht über den Rand des Implantates hinausragen. Damit ist das Implantat leicht in den Raum zwischen die Wirbel einführbar, und es besteht keine Gefahr von Verletzungen der Weichteile oder der Wirbelendplatten. Wenn das Implantat in seiner Stellung zwischen den Wirbeln richtig positioniert ist, werden durch Drehen der Gewindespindel 15 mittels eines Sechskant-Schraubendrehers die beiden keilförmigen Elemente 45, 46 aufeinander zu bewegt, wodurch über die keilförmig gegeneinander geneigten Flächen jeweils eine Kraft auf die schrägen Flächen 63, 64 bzw. 63', 64' des jeweiligen Eingriffsteiles ausgeübt wird und dieses angehoben wird, bis die Zacken 68 über den Rand des Implantates hervorstehen und sich dadurch in dem Wirbelkörper verkrallen können, wie insbesondere in Fig. 2 und Fig. 3 dargestellt ist. Das Ausfahren der Eingriffsteile 60, 61 ist durch den über den Stab 70, 71 gebildeten Anschlag auf eine in Fig. 8 dargestellte Hubhöhe begrenzt, in der die Zacken 68 des jeweiligen Eingriffsteiles über den Rand des Implantates hervorstehen.

Durch das Übersetzen der Drehbewegung des Werkzeugs mittels der Gewindespindel und den keilförmigen Elementen ist eine feindosierte Einstellung des Hubes der Eingriffsteile und damit ein individuelles Anpassen des Implantats an die anatomische Form der Wirbelendplatten des einzelnen Patienten möglich.

Die Ausbildung des Anschlages für die Eingriffsteile 60, 61 in Form des in die zylinderabschnittförmige Ausnehmung 67 eingreifenden Stabes 70, 71 sind die Eingriffsteile 60, 61 in ihrer obersten Stellung drehbar um den jeweilige Stab 70, 71 gelagert. Dies ermöglicht eine selbständige Zentrierung der Eingriffsteile, indem die mit Zacken 68 besetzte Oberfläche der anatomischen Form der jeweiligen Endplatte des Wirbelkörpers folgt. Ein selbständiges Lösen der Eingriffsteile ist aufgrund der Gewindespindel nicht möglich. Nur durch Zurückdrehen mittels des Instrumentes können die Eingriffsteile 60, 61 zurückgefahren werden, wobei der Druck, der von den Wirbeln auf die Eingriffsteile ausgeübt wird, diese in den Implantathohlraum 5 drückt. Damit können die Zacken wieder aus der Wirbelkörperendplatte gelöst werden.

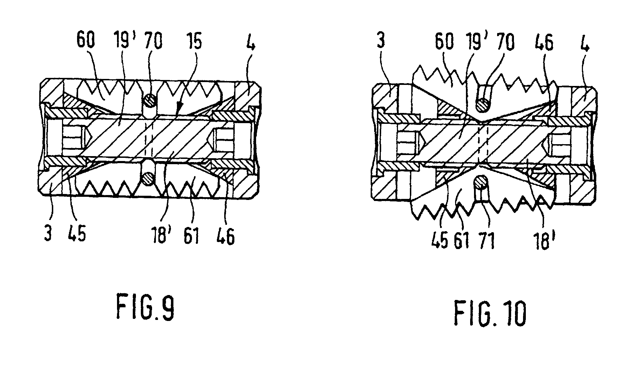

Bei den in den Figuren 1 bis 8 gezeigten ersten Ausführungsbeispielen sind die beiden keilförmigen Elemente mit ihren Keilwinkeln und die beiden jeweils auszufahrenden Elemente mit ihren damit zusammenwirkenden Keilflächen bzw. deren Winkeln so aufeinander abgestimmt, daß die auszufahrenden Elemente mit ihren Oberflächen stets parallel zur Boden- und Deckfläche ausgerichtet sind. Die Ausführungsform ist immer dann gut einsetzbar, wenn die Operation von der Bauchseite her erfolgt, weil der Abstand der benachbarten Wirbelkörper auf der Bauchseite größer als auf der Rückseite ist. Die in den Figuren 9 und 10 gezeigte Ausführungsform ist insbesondere für diejenigen Fälle vorgesehen, bei denen das Element von Rücken her zwischen zwei benachbarte Wirbelkörper eingesetzt werden soll. Für die weitere Beschreibung sind einander entsprechende Teile stets mit dem gleichen Bezugszeichen wie in der ersten Ausführungsform gekennzeichnet.

Bei dem Ausführungsbeispiel sind die Seitenwände rechteckig ausgebildet mit der Folge, daß die Boden- und Deckfläche sich zueinander parallel erstrecken. Wie aus Fig. 9 ersichtlich ist, befinden sich in der Ausgangsstellung die keilförmigen Elemente 45, 46 in der jeweils zu der Vorderwand bzw. Rückwand hin bis zum Anschlag zurückgeschraubten Stellung. Die Elemente 60 und 61 sind dadurch innerhalb des durch die Seitenwände und Vorder- und Rückwand umschlossenen Hohlraumes angeordnet, so daß der Quader, dessen Höhe gleich der Höhe des Spaltes zwischen den benachbarten Wirbeln an seiner engsten Stelle entspricht eingeschoben werden kann.

Abweichend von der ersten Ausführungsform haben die beiden Spindelabschnitte 18', 19' nicht nur einander entgegengesetzte Gewinderichtungen, sondern die Gewindesteigung der beiden Abschnitte ist darüber hinaus unterschiedlich. Dabei ist, wie aus Fig. 10 ersichtlich ist, die Gewindesteigung des Abschnittes 19' um so viel größer vorbestimmt als die Gewindesteigung des Abschnittes 18', daß bei der in Fig. 10 gezeigten Anschlagstellung, an der die Stifte 70, 71 am Grund der U-förmigen Schlitze anliegen, die Elemente 60, 61 mit ihren Oberflächen einen Keilwinkel zueinander bilden, der dadurch entsteht, daß das keilförmige Element 45 aufgrund der größeren Gewindesteigung des Abschnittes 19' bei Drehen der Spindel einen größeren Weg zurücklegt als das Keilelement 46.

Dadurch wird erreicht, daß das in der oben beschriebenen Weise in dem in Fig. 9 gezeigten Zustand eingeschobene Zwischenwirbelimplantat im eingeschobenen Zustand in die in Fig. 10 gezeigte aufgespreizte Stellung bringbar ist, so daß die beiden Elemente 60 und 61 der Spaltneigung zwischen den beiden benachbarten Wirbelkörpern entsprechend aufgespreizt werden und an den beiden Wirbelkörpern anliegen. Dabei ist es nicht erforderlich, daß die Spindel jeweils bis zu Anschlag der Elemente an den Stifte 70, 71 gedreht wird. Auch Zwischenstellungen können gewählt werden.

Abwandlungen der Vorrichtung sind möglich. Beispielsweise können anstatt der Zacken 68 Spitzen oder jede andere Oberflächenstruktur, die ein Eingreifen in Knochenmaterial erlaubt, vorgesehen sein.

## Patentansprüche

1. Zwischenwirbelimplantat mit
zwei einen Abstand zueinander aufweisenden Seitenwänden (1, 2), einer diese an ihrem einen Ende verbindenden Vorderwand (3) und einer dieser gegenüberliegenden Rückwand (4) und je einer Öffnung auf den sich quer zu den vorgenannten Wänden erstreckenden Boden- und Deckflächen, wenigstens einem in dem von den vorgenannten Wänden umschlossenen Raum (5) vorgesehenen Element (60, 61) mit einer zur Deckfläche bzw. Bodenfläche gerichteten Oberfläche, und einem mit dem Element zusammenwirkenden Einstellelement, welches in der Vorderwand und Rückwand gelagert ist, derart, daß das Element zwischen einer ersten Endstellung, in der die Oberfläche nicht über die Boden- bzw. Deckfläche hervorsteht, und einer zweiten Endstellung, in der die Oberfläche über die Boden- bzw. Deckfläche wenigstens teilweise nach außen hervorsteht, hin- und her bewegbar ist.

2. Zwischenwirbelimplantat nach Anspruch 1, **dadurch gekennzeichnet, daß** das Einstellemement in dem von den Wänden umschlossenen Raum (5) eine Gewindespindel (15) mit zwei Abschnitten (18, 19) mit entgegengesetzter Gewindesteigung umfasst, die mit ihrem einen Ende (16) in der Vorderwand und mit ihrem anderen Ende (17) in der Rückwand drehbar gelagert ist und auf der zwei mit ihrer Basis voneinander abgewandte keilförmige Elemente (45, 46) derart gelagert sind, daß beim Drehen der Gewindespindel in eine Richtung die Keilelemente aufeinander zu bewegt werden und beim Drehen der Gewindespindel in eine entgegengesetzte Richtung die Keilelemente voneinander weg bewegt werden und diese Keilelemente auf das Element (60,61) zum Bewegen desselben einwirken.

3. Zwischenwirbelimplantat nach Anspruch 1 oder 2, **dadurch gekennzeichnet, daß** zwei Elemente (60, 61) vorgesehen sind.

4. Zwischenwirbelimplantat nach einem der Ansprüche 1 bis 3, **dadurch gekennzeichnet, daß** die Elemente an ihrer der Oberfläche abgewandten Seite jeweils zwei dachgiebelförmig sich schneidende Flächen (63, 64; 63', 64') aufweisen und daß die Keilelemente mit den schrägen Flächen des jeweiligen Elementes in Eingriff stehen.

5. Zwischenwirbelimplantat nach einem der Ansprüche 1 bis 4, **dadurch gekennzeichnet, daß** die Oberfläche jedes Elementes Zacken (68) zum Eindringen in benachbartes Knochenmaterial aufweist.

6. Zwischenwirbelimplantat nach einem der Ansprüche 1 bis 5, **dadurch gekennzeichnet, daß** ein Anschlag (70, 67; 71, 67') zum Begrenzen der Bewegung jedes Elementes nach außen vorgesehen ist.

7. Zwischenwirbelimplantat nach Anspruch 6, **dadurch gekennzeichnet, daß** der Anschlag durch einen sich im wesentlichen parallel zur Vorder- bzw. Rückwand erstreckenden und in den Seitenwänden getragenen Stift (70; 71) gebildet wird.

8. Zwischenwirbelimplantat nach einem der Ansprüche 1 bis 7, **dadurch gekennzeichnet, daß** die Seitenwände (1, 2) trapezförmig ausgebildet sind, so daß ein keilabschnittsförmiger Körper gebildet ist.

9. Zwischenwirbelimplantat nach einem der Ansprüche 1 bis 8, **dadurch gekennzeichnet, daß** die keilförmigen Elemente unterschiedliche Keilwinkel besitzen.

10. Zwischenwirbelimplantat nach einem der Ansprüche 1 bis 9, **dadurch gekennzeichnet, daß** die beiden Spindelabschnitte unterschiedliche Gewindesteigungen aufweisen.

11. Zwischenwirbelimplantat nach einem der Ansprüche 1 bis 10, **dadurch gekennzeichnet, daß** die Oberfläche der Elemente in ihrer zweiten Stellung eine gegenüber der Boden- bzw. Deckfläche geneigte Stellung aufweisen.

12. Zwischenwirbelimplantat nach einem der Ansprüche 7 bis 11, **dadurch gekennzeichnet, daß** die Elemente jeweils einen sich quer zu ihrer Oberfläche erstreckenden U-förmigen Schlitz aufweisen, in dem jeweils einer der Stifte geführt ist.

## Claims

1. An intervertebral implant having
two mutually spaced side walls (1, 2), a front wall (3) connecting the side walls at their one end, a back wall (4) opposite the front wall, and one opening each at the bottom and top faces extending transversely to the aforementioned walls,
at least one element (60, 61), which is provided in the space (5) enclosed by the aforementioned walls and has a surface directed towards the top face or bottom face, and
an adjusting element, which cooperates with the element and is mounted in the front wall and back wall in such a way that the element is movable to and fro between a first end position, in which the surface does not project beyond the bottom or top face, and a second end position, in which the surface projects at least partly outwards beyond the bottom or top face.

2. An intervertebral implant according to Claim 1, **characterised in that** the adjusting element in the space (5) enclosed by the walls comprises a threaded spindle (15) having two portions (18, 19) with opposite thread pitches, which is rotatably mounted by its one end (16) in the front wall and by its other end (17) in the back wall and on which two wedge-shaped elements (45, 46) with their bases facing away from each other are mounted in such a way that when the threaded spindle is turned in one direction the wedge elements are moved towards each other and when the threaded spindle is turned in an opposite direction the wedge elements are moved away from each other, and these wedge elements act on the element (60, 61) to move it.

3. An intervertebral implant according to Claim 1 or 2, **characterised in that** two elements (60, 61) are provided.

4. An intervertebral implant according to one of Claims 1 to 3, **characterised in that** the elements each have, at their side facing away from the surface, two faces (63, 64; 63', 64') intersecting in the shape of a roof gable, and **in that** the wedge elements are in engagement with the oblique faces of the respective element.

5. An intervertebral implant according to one of Claims 1 to 4, **characterised in that** the surface of each element has teeth (68) for penetrating into adjacent bone material.

6. An intervertebral implant according to one of Claims 1 to 5, **characterised in that** a stop (70, 67; 71, 67') for limiting the outward movement of each element is provided.

7. An intervertebral implant according to Claim 6, **characterised in that** the stop is formed by a pin (70; 71) extending substantially parallel to the front and back walls and supported in the side walls.

8. An intervertebral implant according to one of Claims 1 to 7, **characterised in that** the side walls (1, 2) are trapezoidal, thus forming a body in the shape of a portion of a wedge.

9. An intervertebral implant according to one of Claims 1 to 8, **characterised in that** the wedge-shape elements have different wedge angles.

10. An intervertebral implant according to one of Claims 1 to 9, **characterised in that** the two spindle portions have different thread pitches.

11. An intervertebral implant according to one of Claims 1 to 10, **characterised in that** the surfaces of the elements in their second position are inclined relative to the bottom and top faces respectively.

12. An intervertebral implant according to one of Claims 7 to 11, **characterised in that** the elements each have a U-shaped slot which extends transversely to their surface and in each of which one of the pins is guided.

## Revendications

1. Implant intervertébral comprenant deux parois latérales à distance l'une de l'autre (1, 2), une paroi avant (3) reliant celles-ci à l'une de leurs extrémités et une paroi arrière (4) opposée à celle-ci, et respectivement une ouverture sur laquelle, s'étendent transversalement aux parois ci-dessus des surfaces de plancher et de plafond, au moins un élément (60, 61) prévu dans l'espace (5) entouré par les parois citées ci-dessus, avec une surface orientée vers la surface de plafond ou la surface de plancher, et un élément de positionnement interagissant avec l'élément, l'élément de positionnement étant placé dans la paroi avant et la paroi arrière de telle sorte que l'élément puisse être déplacé dans un sens ou dans un autre, entre une première position finale dans laquelle la surface ne dépasse pas au-dessus de la surface de plafond ou de la surface de plancher, et une deuxième position finale, dans laquelle la surface dépasse sur l'extérieur au moins en partie au-dessus de la surface de plafond ou de la surface de plancher.

2. Implant intervertébral selon la revendication 1, **caractérisé en ce que** l'élément de positionnement dans l'espace (5) entouré par les parois comprend une broche filetée (15) dotée de deux tronçons (18, 19) avec un pas de vis inversé, dont une des extrémités (16) est placée de façon à pouvoir tourner dans la paroi avant et l'autre extrémité (17), de façon à pouvoir tourner dans la paroi arriére et sur laquelle deux éléments en coin (45, 46) détournés l'un de l'autre avec leur base, sont placés de telle sorte qu'en tournant la broche filetée dans une direction, les éléments en coin puissent être déplacés l'un vers l'autre et qu'en tournant la broche filetée dans une direction opposée, les éléments en coin puissent être déplacés en s'éloignant l'un de l'autre et ces éléments en coin agissant sur l'élément (60, 61) pour déplacer ceux-ci.

3. Implant intervertébral selon la revendication 1 ou 2, **caractérisé en ce que** deux éléments (60, 61) sont prévus.

4. Implant intervertébral selon l'une quelconque des revendications 1 à 3, **caractérisé en ce que** les éléments présentent sur leur côté détourné de la surface, respectivement deux surfaces se coupant en pignon de toit (63, 64 ; 63', 64') et que les éléments en coin sont en prise avec les surfaces obliques des éléments respectifs.

5. Implant intervertébral selon l'une quelconque des revendications 1 à 4, **caractérisé en ce que** la surface de chaque élément présente des dents (68) pour pénétrer dans le matériau osseux adjacent.

6. Implant intervertébral selon l'une quelconque des revendications 1 à 5, **caractérisé en ce qu'**une butée (70, 67 ; 71, 67') est prévue pour limiter le mouvement de chaque élément vers l'extérieur.

7. Implant intervertébral selon la revendication 6, **caractérisé en ce que** la butée est formée par une tige (70, 71) s'étendant essentiellement parallèlement à la paroi avant et à la paroi arrière et étant portée dans les parois latérales.

8. Implant intervertébral selon l'une quelconque des revendications 1 à 7, **caractérisé en ce que** les parois latérales (1, 2) sont constituées en forme de trapèze de sorte qu'un corps en forme de partie de coin soit formé.

9. Implant intervertébral selon l'une quelconque des revendications 1 à 8, **caractérisé en ce que** les éléments en forme de coin possèdent des angles de coin différents.

10. Implant intervertébral selon l'une quelconque des revendications 1 à 9, **caractérisé en ce que** les deux tronçons de broche présentent des filetages différents.

11. Implant intervertébral selon l'une quelconque des revendications 1 à 10, **caractérisé en ce que** la surface des éléments présente, dans leur deuxième position, une position penchée en face de la surface de plafond ou de la surface de plancher.

12. Implant intervertébral selon l'une quelconque des revendications 7 à 11, **caractérisé en ce que** les éléments présentent respectivement une fente en forme de U s'étendant perpendiculairement à leur surface, dans laquelle respectivement l'une des tiges est insérée.
